# EUROPEAN PATENT APPLICATION

(11) **EP 4 768 047 A1**
(43) Date of publication of application: **01.07.2026**
(21) Application number: 25227802.3
(22) Date of filing: 30.12.2025
(51) Int. Cl.: A61L 9/20, F24F 8/22

(54) **UV-C MICROBIOLOGICAL INACTIVATION APPARATUS FOR AIR CONDITIONING AND VENTILATION GRILLES**

(30) Priority: 30.12.2024 PT 2024119968
(71) Applicant: Oprimee - Innovation Design Engineering Solutions, Lda, 3405-155 Oliveira do Hospital (PT)
(72) Inventor: DOS SANTOS ALMEIDA NUNES, João Miguel, 3405-155 Oliveira do Hospital (PT)
(74) Representative: Patentree

(57) **Abstract**

The present invention describes a microbiological inactivation system using UV-C radiation, designed to be integrated into air conditioning and ventilation grilles, aiming to improve indoor air quality and minimizing the presence of pathogenic microorganisms such as viruses, bacteria and fungi. The system comprises a microbiological inactivation module with UV-C lamps arranged in a sinuous circuit, which maximizes the air's exposure to ultraviolet radiation and the reflection and redistribution of UV-C radiation by internal surfaces. In addition, the blower is equipped with an electric motor controlled by a frequency inverter, allowing bidirectional operation of the fan (air insufflation and extraction), which provides flexibility in ventilating and purifying the environment. Installation and maintenance are made easy thanks to the design, which allows the system to be adapted to different air volumes and microbiological control needs, without the need for complex modifications to existing air conditioning and ventilation. This microbiological inactivation system using UV-C radiation contributes to improving air quality, increasing microbiological safety in different contexts.

## Description

### TECHNICAL FIELD

The present disclosure relates to a UV-C microbiological inactivation apparatus for air conditioning and ventilation grilles.

### BACKGROUND

HVAC (Heating, Ventilation, and Air Conditioning) systems are widely used in multiple contexts, ranging from residential to commercial and industrial environments, to ensure proper control of temperature, humidity and, above all, air quality. These systems are responsible for capturing ambient air, processing it through heating, cooling or filtering, and recirculating it in the treated space. Although thermal efficiency and humidity are the most discussed and optimized functions in HVAC systems, indoor air quality has become a growing concern, especially regarding pathogenic microorganisms and other pollutants in the air.

In recent years, with the increase in airborne diseases such as flu, colds and, more recently, the COVID-19 pandemic, there has been a growing global awareness of the importance of indoor air quality and the need for systems capable of reducing or eliminating pathogens present in the environment. Continuous exposure to these pathogens can pose serious health risks, particularly in closed environments with high population density, such as hospitals, schools, commercial offices and industries. Although traditional HVAC systems have air filters to remove large particles such as dust and allergens, they are not effective at eliminating microorganisms such as viruses, bacteria and fungi that may be present in recirculated air.

In addition, conventional HVAC systems, made up of rotors that manage air circulation, are not designed to deal with microbiological contamination of the air. This means that, in many cases, contaminated air can be recirculated within the system, compromising indoor air quality and putting occupants' health at risk. Another challenge with traditional blowers is that their casings and ducts are often not suitable for blocking the recirculation of contaminated air, which can lead to ineffective distribution of purified air and a greater likelihood of pathogens spreading. In particular, existing solutions often rely on filtration or isolated UV-C exposure stages that do not guarantee a controlled accumulated UV-C dose across the entire airflow, especially when airflow velocity, turbulence or operating regimes vary.

The solutions currently available on the market air treatment solutions for HVAC applications are frequently implemented as linear in-duct irradiation sections or auxiliary purification units, such as external air purification systems or large UV-C filters, often require significant modifications to the HVAC system or additional installations, which can considerably increase the cost and complexity of implementation. These systems may also not be as effective or may be difficult to maintain on a large scale, especially in environments with HVAC blowers already installed, such as those in old buildings or those that don't have the infrastructure to incorporate complex purification solutions.

In addition, maintenance and adjustment of conventional HVAC blowers, especially those used in environments with high air volumes, can be a challenge due to the lack of easy access to the internal components or the lack of modular solutions that allow the system to be customized according to the specific microbiological purification needs of each environment.

Furthermore, many existing air disinfection systems depend primarily on increasing UV-C power or lamp intensity rather than on controlling the residence time of the air within the irradiation zone. This approach can lead to inefficient energy use, non-uniform microbiological inactivation and increased safety constraints, particularly when applied to retrofitted or compact HVAC installations.

As a result, there is a need for an integrated solution that enables effective microbiological inactivation of circulating air within existing HVAC systems, without the need for complex structural changes. A solution that attaches directly to existing blowers can provide simple installation, easy maintenance and, most importantly, a significant improvement in indoor air quality, guaranteeing healthier and safer environments.

These facts are disclosed in order to illustrate the technical problem addressed by the present disclosure.

### GENERAL DESCRIPTION

The present disclosure relates to a UV-C microbiological inactivation apparatus for air conditioning and ventilation grilles.

The present disclosure comprises a microbiological inactivation apparatus for HVAC systems, configured as a stationary module installable in-line or in bypass within an HVAC duct, the apparatus comprising: one or more UV-C radiation sources configured to emit ultraviolet radiation in a wavelength range effective for microbiological inactivation, preferably at wavelengths from 190-250 nm; an enclosed internal treatment volume defining an airflow path for HVAC air, the airflow path comprising a labyrinthine or sinuous internal geometry having a cumulative internal flow length greater than the external longitudinal length of the module, such that the residence time of the air within the treatment volume is increased relative to a straight-through duct section; wherein the internal geometry is dimensioned such that, for a predefined operating range of HVAC volumetric airflow rates, the air residence time within the treatment volume is sufficient to deliver a predetermined minimum accumulated UV-C dose to the circulating air; and at least one airflow-driving mechanism configured to cooperate with the HVAC system to convey air through the treatment volume, wherein the apparatus is configured to operate without physical displacement of the UV-C sources or the module during operation.

The disclosed technology does not reside merely in the presence of UV-C radiation, but in the controlled application of a minimum accumulated UV-C dose achieved through residence time determined by internal geometry and HVAC airflow conditions.

The present disclosure relates to a microbiological inactivation apparatus integrated into existing HVAC systems, designed to improve indoor air quality by eliminating pathogens through the use of UV-C radiation. The apparatus comprises one or more UV-C lamps emitting radiation in the range of 190-250 nm and/or 253-255nm, which effectively inactivate harmful microorganisms in the circulating air.

In an embodiment, the apparatus is equipped with an electric motor controlled by a frequency inverter, allowing the blower to operate in a bidirectional manner for both air intake and extraction, depending on the environmental needs. This provides flexibility in air circulation and purification.

Designed for easy installation into conventional HVAC systems, the now disclosed technology helps enhance the safety and health of indoor environments by reducing the presence of airborne pathogens. The apparatus can operate continuously or on-demand, adapting to different airflow and microbiological control requirements.

The present technology relates to a microbiological inactivation apparatus with UV-C radiation, designed to be integrated into HVAC grills, with the aim of improving indoor air quality and eliminating microbiological pathogens during air circulation.

In an embodiment, the fitting apparatus comprises a microbiological inactivation module that includes at least one UV-C lamp, with wavelengths from 190-250 nm, preferably from 253-255 nm, responsible for inactivating pathogenic microorganisms present in the air as it passes through the HVAC systems, thereby reducing the spread of microorganisms, such us, viruses, bacteria, and fungi and others in the environment.

In an embodiment, the apparatus comprises an electric motor controlled by a frequency inverter, allowing the blower to operate in a bidirectional manner, enabling both air intake and extraction depending on the needs of the environment.

In an embodiment, the apparatus comprises a modular design, which allows for easy installation in existing HVAC systems without requiring significant modifications to the system's infrastructure, also facilitating maintenance and adjustments according to the microbiological purification requirements of each environment.

In an embodiment, the UV-C microbiological inactivation apparatus is designed to operate continuously or on-demand, allowing the air flow to be adjusted according to the required air volume and the intensity of microbiological control needed in the space.

In an embodiment, the fitting apparatus is characterized by its energy efficiency, providing a sustainable solution for air purification and pathogen reduction, while maintaining the operational efficiency of the HVAC systems, valuing the reflection and redistribution of UV-C radiation by internal surfaces by the type of equipment configuration, originated by the type of material and type and finish of material surface, for example, polished zinc and polished stainless steel 304, or 316, 316L, and geometric shape.

This innovative design of a UV-C microbiological inactivation apparatus is particularly beneficial for residential, commercial, industrial, and healthcare environments, where indoor air quality and the reduction of microbiological risks are crucial for the health and safety of occupants.

It is disclosed a microbiological inactivation apparatus for HVAC systems, comprising: one or more UV-C lamps configured to emit ultraviolet radiation at wavelengths from 190-250 nm; a sinuous circuit configured to increase the exposure time of air to UV-C radiation for at least 5 seconds; and a bidirectional extractor equipped with an electric motor controlled by a frequency inverter, enabling air insufflation and extraction.

It is disclosed a microbiological inactivation apparatus for HVAC systems for delivering a predetermined minimum accumulated UV-C dose to circulating air, configured as a stationary module installable in-line or in bypass within an HVAC duct, the apparatus comprising: a grille; one or more UV-C radiation sources configured to emit ultraviolet radiation in a wavelength range effective for microbiological inactivation; an enclosure defining an internal treatment volume for an airflow path for HVAC air; wherein the enclosure comprises an expanding channel comprising a plurality of baffles offset from one another along the expanding direction of the channel for defining a meandering internal airflow geometry; wherein the meandering internal airflow geometry has a cumulative internal flow length greater than the external longitudinal length of the module; wherein the expanding channel is inclined in relation to the grille.

Preferably the inclination of the expanding channel forms a degree of forms an angle of 10° to 45°, preferably 15° to 35°, from the grille..

Preferably the plurality of baffles are interleaved, arranged so as to define a meandering airflow path. More preferably the baffles are offset from one another along the expanding direction of the channel, particularly from the grille to the bidirectional extractor.

In an embodiment, each UV-C radiation source is arranged between two adjacent offset baffles.

In an embodiment, the enclosure defines an internal treatment volume for an airflow path for HVAC air, such that the residence time of the air within the treatment volume is increased relative to a straight-through duct section.

In an embodiment, the internal treatment volume geometry is dimensioned such that, for a predefined operating range of HVAC volumetric airflow rates, the air residence time within the treatment volume is sufficient to deliver a predetermined minimum accumulated UV-C dose to the circulating air.

In an embodiment, the predetermined minimum accumulated UV-C dose is at least 20 mJ/cm², calculated as a function of effective irradiance and air residence time.

In an embodiment, the cumulative internal flow length of the airflow path is at least 1.5 times the external longitudinal length of the module.

In an embodiment, the internal treatment volume is optically enclosed such that UV-C irradiance measured outside the module remains below a predefined safety threshold during operation.

In an embodiment, the apparatus is configured to operate with a pressure loss not exceeding a predefined percentage of the nominal HVAC system pressure.

In an embodiment, at least one airflow-driving mechanism is configured to cooperate with the HVAC system to convey air through the treatment volume, preferably the airflow-driving mechanism is a blower.

In an embodiment, the apparatus is configured to operate without physical displacement of the UV-C sources or the module during operation.

In an embodiment, the sinuous circuit of the microbiological inactivation apparatus is configured as a serpentine or meandering path to maximize the air's exposure to UV-C radiation, ensuring enhanced inactivation of airborne pathogens while optimizing energy consumption

In an embodiment, the UV-C lamps of the microbiological inactivation apparatus are positioned within the airflow pathway of the sinuous circuit to ensure multiple passes of the air through the UV-C radiation zone for increased disinfection efficiency.

In an embodiment, the bidirectional extractor of the microbiological inactivation apparatus enables: insufflation, directing air from the environment into the HVAC system for conditioning and purification; and extraction, removing contaminated air from the indoor environment for treatment.

In an embodiment, the frequency inverter of the microbiological inactivation apparatus allows precise control of the motor speed of the bidirectional extractor, optimizing air circulation and energy efficiency based on environmental requirements.

In an embodiment, the UV-C lamps and motor of the microbiological inactivation apparatus are configured to minimize energy consumption while maintaining effective microbiological inactivation and air circulation.

In an embodiment, the microbiological inactivation apparatus is configured for continuous operation to provide ongoing microbiological disinfection of air or on-demand activation based on air quality sensors or user preferences.

In an embodiment, the microbiological inactivation module of the microbiological inactivation apparatus is configured to integrate seamlessly into existing HVAC grills without requiring significant modifications to the blower or ductwork.

In an embodiment, the sinuous circuit of the microbiological inactivation apparatus optimizes the use of space within the HVAC grill to accommodate high air volumes while ensuring effective pathogen inactivation.

In an embodiment, the UV-C lamps of the microbiological inactivation apparatus inactivate airborne pathogens, allergens, and pollutants, contributing to improved indoor air quality in environments such as hospitals, schools, offices, and manufacturing plants.

In an embodiment, the UV-C lamps of the microbiological inactivation apparatus are enclosed within a protective housing to prevent harmful UV-C radiation from escaping the inactivation module, ensuring operator and environmental safety.

In an embodiment, the external structure of the microbiological inactivation apparatus is compact and modular, allowing for easy installation and maintenance within existing HVAC units.

In an embodiment, the bidirectional blower of the microbiological inactivation apparatus adapts to environmental needs by selectively operating in: intake mode for drawing air into the HVAC system for purification; and exhaust mode for removing contaminated air from the indoor environment.

In an embodiment, the arrangement of the UV-C lamps within the sinuous circuit in the microbiological inactivation apparatus ensures uniform and prolonged exposure of air to UV-C radiation for optimal pathogen inactivation.

In an embodiment, the microbiological inactivation apparatus further comprises air quality sensors configured to monitor indoor air conditions and automatically activate the UV-C lamps and extractor based on predefined thresholds for contaminants or pathogens.

### BRIEF DESCRIPTION OF THE DRAWINGS

The following figures provide preferred embodiments for illustrating the disclosure and should not be seen as limiting the scope of invention.
**Figure** 1: Schematic representation of an embodiment of a microbiological inactivation apparatus for HVAC systems.
**Figure 2****:** Schematic representation of the assembly view of the microbiological inactivation apparatus for HVAC systems

### DETAILED DESCRIPTION

The present disclosure relates to a UV-C microbiological inactivation apparatus for air conditioning and ventilation grilles.

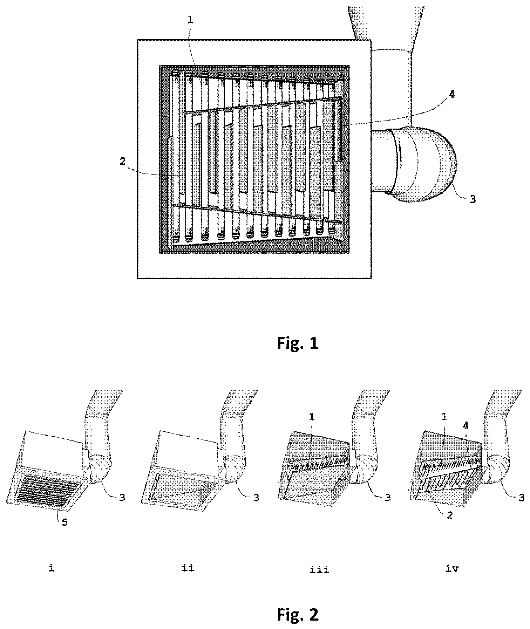

The present technology relates to a microbiological inactivation apparatus for HVAC, represented in Fig. 1, designed to improve indoor air quality by removing harmful microorganisms such as viruses, bacteria, and fungi through the use of UV-C radiation. The apparatus is integrated directly into existing HVAC grills, providing a simple and efficient solution for enhancing air safety in different environments.

The core component of the now disclosed technology is a UV-C inactivation module. This module comprises one or more UV-C lamps (1) emitting ultraviolet radiation at wavelengths from 190-250 nm, preferably from 253-255 nm and an exposition time over 5 seconds. This specific wavelength range is highly effective at inactivating microorganisms by damaging their DNA and RNA, preventing them from replicating and rendering them harmless.

In an embodiment, the UV-C lamps are positioned within an airflow pathway designed as a sinuous circuit and the maximization refraction radiation improving the efficiency inactivation and decrease the energy consumptions, and density energy and irradiation of the UV-C (2). The sinuous circuit is a unique feature of the invention, engineered to maximize the exposure of the circulating air to UV-C radiation over 5 seconds. As the air travels through the circuit, it follows a winding or serpentine path that increases the time the air is exposed to the UV-C lamps, with an important process because is necessary to ensure the decay of microorganism. This extended exposure time ensures more effective inactivation of airborne pathogens before the air is returned to the indoor space.

The sinuous circuit not only enhances pathogen inactivation but also optimizes the space within the HVAC grills, ensuring that the UV-C lamps can treat larger volumes of air efficiently. The circuit is designed to fit seamlessly into the airflow path, allowing for both high air volume processing and effective pathogen removal.

To provide flexibility in air circulation, the bidirectional extractor (3) is equipped with an electric motor that is controlled by a frequency inverter. This motor allows the blower to operate in two directions, insufflation (intake), in which the motor directs the airflow from the environment into the HVAC system, allowing the air to be conditioned and purified before being returned to the indoor space, through the air inlet/outlet (4); and extraction, in which the motor draws air from the indoor environment for treatment, ensuring that contaminated air is removed and replaced with purified air. This bidirectional functionality allows the apparatus to adapt to various environmental needs, such as circulating clean air or reducing contaminants, depending on the requirements of the space.

The combination of the sinuous circuit and the UV-C lamps creates a highly efficient air purification apparatus. The air is forced to travel along a winding path, allowing it to pass through the UV-C radiation multiple times as it circulates, ensuring thorough disinfection and purification of the air before it is released back into the environment. The apparatus is designed to ensure that the air spends a sufficient amount of time in the UV-C zone, increasing the likelihood of pathogen inactivation.

In an embodiment, the apparatus provides continuous microbiological disinfection of the air, helping to reduce the spread of airborne diseases and improving overall air quality. The apparatus can operate continuously to ensure constant air purification or on-demand, activating based on air quality sensors or user preferences.

One of the key advantages of this technology is its ability to integrate seamlessly into existing HVAC systems. The apparatus is designed to be easily installed without requiring extensive modifications to the existing blower or ductwork. It can be retrofitted to most standard HVAC systems, making it a cost-effective solution for enhancing indoor air quality without the need for complete apparatus replacements.

The integration process involves connecting the UV-C inactivation module and motor control system to the blower, allowing them to work in conjunction with the existing air filtration and conditioning components of the HVAC system.

In an embodiment, the apparatus is designed with energy efficiency in mind. The UV-C lamps and motor are selected to ensure minimal energy consumption while maintaining optimal disinfection and airflow performance. The frequency inverter allows for precise control over the blower's motor speed, optimizing energy use based on the air volume and the needs of the space.

By incorporating UV-C radiation into HVAC systems, this technology offers significant health and safety benefits. It helps reduce the concentration of airborne pathogens, allergens, and pollutants, contributing to a healthier indoor environment. This is especially important in environments where air quality is critical, such as hospitals, schools, offices, and manufacturing plants.

The apparatus is particularly valuable in places where airborne diseases can spread rapidly and efficiently, as the UV-C radiation inactivates viruses and bacteria, preventing their transmission through the air. This makes it an ideal solution for improving the overall air hygiene in indoor spaces and mitigating the risks associated with indoor air pollution.

Figure 1 illustrates the general concept of the technology, that relates to a microbiological inactivation apparatus for HVAC systems, positioned in the grills of extraction and insufflation air for indoor buildings, comprising uv-c lamps (1) for microbiological inactivation, a winding circuit structure (2) to increase the exposure time of the air to the treatment of the UV-C radiation and a bidirectional extractor (3), which allows the circulation of the treated air through the air inlet/ outlet (4).

Fig. 2 presents the assembly view of the microbiological inactivation apparatus for HVAC blowers, with different angles and details of the equipment. The description covers the various provided views, revealing the operation and both internal and external structure of the apparatus. The external view (i) of the microbiological inactivation apparatus for HVAC shows the complete device, highlighting the outer casing and the main visible components. The equipment is designed to be integrated directly into HVAC blowers, featuring a robust and functional design. The external structure is compact, allowing installation in existing HVAC units without major modifications. The view without the lower grille (5) (ii) shows the external part of the structure of the sinuous circuit (2), as well as the bidirectional blower (3). The view without the upper cover (iii) allows observation of the UV-C lamps (1) installed inside the inactivation module, ensuring that the air passing through them receives the necessary UV-C radiation for the destruction of viruses, bacteria, and fungi. The complete internal view (iv) of the apparatus reveals the main components of the microbiological inactivation apparatus, such as the UV-C lamps (1), the sinuous circuit (2), and the bidirectional blower (3). The interior of the equipment shows the arrangement of the UV-C lamps (1) along the sinuous circuit, ensuring that the air is effectively exposed to UV-C radiation during its path.

Fig. 2 shows the assembly view of the microbiological inactivation apparatus for HVAC systems, taking into account the external view of the equipment (i), the internal view without the lower grille (5) (ii), the view without the upper cover (iii) and the internal view (iv).

In order to ensure effective microbiological inactivation, the apparatus is configured to guarantee a predefined minimum accumulated UV-C dose within the internal treatment volume. The UV-C dose is expressed as energy per unit area, measured in millijoules per square centimetre, and corresponds to the minimum dose required to inactivate targeted microorganisms under worst-case operating conditions. The applied dose is determined as a function of the effective irradiance incident on the treated air and internal surfaces, expressed in milliwatts per square centimetre, and the effective exposure time during which the air resides within the UV-C radiation field. The relationship between accumulated dose, effective irradiance and exposure time follows the relation D = E × t, where D is the dose in mJ/cm², E is the effective irradiance in mW/cm² and t is the exposure time in seconds. The effective irradiance already incorporates optical, geometric and environmental losses. The apparatus is configured such that the minimum target dose is achieved for all permitted operating conditions, including maximum airflow velocity, maximum distance between the UV-C sources and the treated air, and foreseeable partial occlusions.

The effective irradiance considered by the system incorporates correction factors accounting for the distance between the UV-C sources and the irradiated volume, angular incidence of radiation, partial shading caused by internal geometry or turbulent flow, attenuation due to aerosols or suspended particles, internal surface reflectivity, and temporal degradation of the UV-C sources over their operational lifetime. Internal surfaces of the treatment volume may be formed from UV-resistant and UV-reflective materials, including aluminium, stainless steel, galvanised steel, zinc-coated steel or metallised polymers, selected to enhance irradiation uniformity and compensate for optical losses while maintaining durability under prolonged UV-C exposure.

In an embodiment, the apparatus defines an enclosed internal treatment volume configured as a tunnel or capsule through which the airflow of the HVAC system passes. The treatment volume is characterised by a useful width from 0.3 m to 3.0 m, a useful height from 0.3 m to 4.0 m, and a nominal longitudinal length from 0.5 m to 5.0 m. Within this external envelope, the internal airflow path is shaped to form a labyrinthine or sinuous circulation path whose cumulative internal flow length exceeds approximately 8.0 m, thereby increasing the residence time of the air within the UV-C radiation field without requiring an increase in the external dimensions of the module. This configuration allows sufficient contact time between UV-C radiation and microorganisms to ensure decay and inactivation.

The internal geometry further comprises inlet and outlet transition zones configured to ensure complete optical confinement of the UV-C radiation and to prevent leakage towards the exterior of the capsule. The internal layout explicitly defines a treatment volume, within which the minimum UV-C dose is guaranteed, and a containment volume, which may extend beyond the treatment volume and serves to ensure radiological safety and aerodynamic stability. These volumes may be geometrically distinct and functionally complementary.

The UV-C sources are distributed within the capsule in a multi-angular arrangement, including lateral, upper, frontal, rearward and optionally lower orientations, such that multiple irradiation fields overlap within the treatment volume. This overlapping configuration mitigates shadowing effects caused by airflow turbulence, particles or internal structural elements and promotes uniform dose distribution across the entire airflow cross-section, including under conditions of non-laminar or turbulent flow.

The effective exposure time of the air to UV-C radiation is defined exclusively by the internal geometry of the capsule and the volumetric airflow rate of the HVAC system. The exposure time corresponds to the residence time of the air within the treatment volume and is determined by the ratio between the internal useful volume of the capsule and the volumetric airflow rate. As the apparatus is stationary and integrated into the HVAC ductwork, no mechanical displacement of the capsule or the UV-C sources is involved in determining the exposure time. The system is configured such that, across the entire permitted operating range of airflow rates, the residence time remains sufficient to ensure delivery of the predefined minimum UV-C dose.

The apparatus is configured to operate in conjunction with the existing airflow regime of the HVAC system while maintaining controlled aerodynamic conditions within the capsule. Relevant airflow parameters include air velocity, volumetric flow rate, differential pressure between the interior and exterior of the capsule, and airflow direction. Depending on the application, the airflow may be longitudinal, transverse, descending or configured as an air curtain. In certain embodiments, the capsule operates under a slight negative internal pressure relative to the surrounding ductwork, thereby ensuring confinement of irradiated air and preventing escape of aerosols, ozone or partially treated air. The internal geometry and airflow control are dimensioned to minimise dead zones, recirculation pockets and particle re-entrainment, thereby improving treatment uniformity and reducing the risk of recontamination.

The apparatus incorporates an intrinsically safe architecture ensuring that UV-C radiation is fully confined within the capsule during operation. The enclosure is designed to be optically and mechanically sealed through the use of overlapping panels, labyrinth seals, gaskets and UV-opaque materials, such that no direct or indirect UV-C radiation escapes to the exterior. The irradiance measured outside the capsule remains below predefined safety thresholds at specified distances from the external surface. Electrical and mechanical interlocks are provided such that any opening of access panels or structural elements results in immediate interruption of UV-C emission. Presence detection sensors may be employed to detect human proximity or intrusion, triggering an immediate transition to a safe state. The apparatus is thereby suitable for continuous operation in occupied environments, with redundancy provided through multiple independent safety layers.

In an embodiment, the apparatus may further comprise a control system implementing a closed-loop control algorithm configured to regulate UV-C output, airflow parameters and safety functions. Input variables may include airflow velocity, volumetric flow rate, differential pressure, internal irradiance measurements, temperatures of UV-C sources and electronic components, interlock states and presence detection signals. In an embodiment, output variables may include UV-C source power modulation, fan or damper control, alarm signalling and event logging. The control algorithm dynamically adjusts UV-C output to ensure that the accumulated dose delivered to the air meets or exceeds the predefined minimum dose for the estimated or measured residence time, while safety-related rules are assigned highest priority such that any violation results in immediate deactivation of UV-C emission and transition to a safe operating mode. Operational data may be recorded to provide traceability and verification of system performance.

Typical experimental parameters applicable to stationary UV-C treatment capsules integrated into HVAC systems include ultraviolet wavelengths in the range of 190 to 250 nm and/or 253 to 255 nm when using mercury-based UV-C lamps, or alternatively in the range of 265 to 280 nm when using UV-C LED sources. The effective UV-C irradiance within the internal treatment volume typically lies from 0.2 mW/cm² to 10 mW/cm², while the accumulated UV-C dose delivered to the air and internal surfaces typically ranges from 20 mJ/cm² to 120 mJ/cm². The residence time of the air within the capsule may vary from 0.5 seconds to 120 seconds, depending on the volumetric airflow rate and the internal geometry of the capsule. Typical HVAC airflow rates range from 100 m³/h to 5 000 m³/h, with an internal-to-external differential pressure maintained from -5 Pa to -50 Pa. The average air velocity within the capsule typically lies from 0.2 m/s to 2.0 m/s. The geometry of the capsule is configured to be compatible with both circular and rectangular HVAC ducts.

The exposure time of the air to UV-C radiation results exclusively from the residence time of the air within the internal volume of the capsule and is a function of the volumetric airflow rate of the HVAC system and the internal geometry of the capsule. The apparatus is stationary and does not involve any physical displacement or mobility of the UV-C module during operation. In certain embodiments, the apparatus may be configured to allow the addition of an auxiliary ventilation element, where justified, to compensate for pressure losses exceedingly approximately 20 percent caused by the introduction of the capsule into the HVAC system, while preserving the required residence time and UV-C dose.

Tests conducted under controlled conditions representative of operation in HVAC systems demonstrate that the multi-angular arrangement of the UV-C sources provides a high degree of dose uniformity within the internal treatment volume, including under turbulent airflow regimes. The residence time of the air within the capsule is sufficient to guarantee application of the target UV-C dose without requiring an increase in the external length of the capsule. Operation with controlled or slightly negative internal pressure reduces downstream release of aerosols and particulate matter within the HVAC system. Closed-loop control of the UV-C irradiance compensates for variations in airflow rate, ageing of the UV-C sources and aerodynamic fluctuations of the HVAC system. Measurements further confirm that UV-C irradiance outside the capsule remains below established safety limits, enabling continuous operation in occupied environments as a result of the encapsulation principle.

Proof-of-concept activities and laboratory tests were conducted to demonstrate that the stationary UV-C capsule integrated into HVAC systems is capable of effectively applying a predefined target UV-C dose to circulating air and exposed internal surfaces under operating conditions representative of HVAC use. The tests further aimed to confirm that the apparatus maintains complete optical confinement of UV-C radiation and, where applicable, aerodynamic confinement through controlled pressure differentials and internal airflow management, while ensuring full safety for human occupants by maintaining external irradiance levels below established safety limits and by means of redundant interlock mechanisms. In addition, the tests demonstrated that the system allows dynamic and repeatable control of treatment efficacy, compensating for variations in airflow rate, ageing of UV-C sources and operational fluctuations of the HVAC system. These tests are not intended to limit the scope of the invention but to substantiate its technical feasibility and the technical effects achieved by the claimed combination in an HVAC context.

During the tests, a set of critical independent parameters was defined and monitored. Ultraviolet radiation was generated at wavelengths typically around 254 nm using mercury-based UV-C lamps, or alternatively in the range of 265 to 280 nm using UV-C LED sources, and in certain configurations within the far UV-C range from 204 to 218 nm. The effective UV-C irradiance within the internal treatment volume was varied typically from 0.3 mW/cm² to 8.0 mW/cm², resulting in accumulated UV-C doses from 20 mJ/cm² to 120 mJ/cm². Spatial dose uniformity within the treatment volume was maintained within a deviation range of approximately ±10 to ±25 percent, with source-to-target distances within the capsule ranging from 0.2 m to 1.0 m. Measurement of irradiance was performed by calibrated UV-C radiometry at multiple points within the useful treatment volume, complemented by passive UV-C dosimeters placed in critical regions corresponding to maximum distance, partial shading and low-irradiance zones.

Exposure conditions representative of HVAC operation were established by varying the volumetric airflow rate from 100 m³/h to 5 000 m³/h, resulting in air residence times within the capsule ranging from 0.5 seconds to 120 seconds as a function of the internal geometry. Validation confirmed that, for the entire tested airflow range, the capsule ensured the delivery of at least the predefined target UV-C dose, with the exposure time being defined exclusively by the residence time of the air within the internal useful volume. No physical movement or displacement of the UV-C capsule or radiation sources was involved, the relevant dynamics being solely those of the airflow within the HVAC system.

Aerodynamic compatibility with HVAC operation was assessed by monitoring airflow rate, average air velocity within the useful cross-section, internal-to-external pressure differential and pressure losses introduced by the capsule. Average air velocities typically ranged from 0.2 m/s to 2.0 m/s, while differential pressure, when applied, was maintained from -5 Pa to -40 Pa. Flow visualisation using tracer smoke and neutral particles confirmed the absence of undesirable bypass paths and critical internal recirculation zones, as well as acceptable pressure losses compatible with typical HVAC operating regimes. Where applied, controlled negative internal pressure was shown to contribute to effective containment of aerosols within the capsule.

Safety-related parameters were verified by measuring external UV-C irradiance at defined distances from the capsule, confirming values below 0.001 mW/cm². Interlock response times following detection of panel opening, intrusion or unsafe conditions were measured to be below 100 milliseconds, with at least two independent safety systems operating redundantly. Failure tests included deliberate opening of access panels during operation, simulation of maintenance intervention, partial ventilation failure or airflow outside defined limits, and failure of primary sensors with automatic transition to a safe state.

Efficacy tests performed under conditions representative of HVAC operation included microbiological assays using indicator bacteria, model viruses, fungi and spores, as well as surface-associated biofilms representative of internal HVAC surfaces. Observed results included logarithmic reductions typically ranging from three to six orders of magnitude for UV-C doses from 40 mJ/cm² to 100 mJ/cm², with consistent efficacy under representative airflow conditions, including regions of disturbed or turbulent flow. Reproducibility across test cycles exceeded approximately 95 percent.

Additional tests were conducted using geometries and materials representative of HVAC installations, including metallic and polymeric surfaces commonly used in ducts and air handling units, materials with differing UV reflectance coefficients, and internal elements simulating grilles, deflectors and technical obstacles. These tests confirmed that the combined effect of multi-angular UV-C source arrangement, optimised internal geometry and closed-loop control of irradiance or source power reduces zones of sub-exposure and improves robustness of treatment against variations in airflow and operating conditions typical of HVAC systems.

The results of the proof-of-concept and laboratory tests demonstrate that the technical effects achieved by the invention do not arise solely from the application of UV-C power, but from the functional integration of accumulated dose control, air residence time determined by internal geometry, controlled aerodynamics and safety-oriented encapsulation within an HVAC-compatible stationary capsule. Known UV-C systems for HVAC applications typically fail to address at least one of these critical aspects, such as dose uniformity, aerodynamic compatibility or optical confinement and human safety. The experimentally demonstrated effect, namely the reproducible application of a predefined minimum UV-C dose to circulating HVAC air under variable airflow conditions while maintaining optical confinement, aerodynamic compatibility and human safety, is therefore not obvious to a person skilled in the art and confirms both the technical feasibility and the inventive contribution of the claimed invention.

By virtue of these characteristics, the invention simultaneously addresses the problem of achieving effective and uniform UV-C dose application in stationary HVAC systems, the risk of accidental human exposure to UV-C radiation in occupied environments, and the persistence of microbiological and particulate contamination associated with airflow dynamics in HVAC ductwork. These technical effects are achieved without a significant increase in pressure losses, without degradation of the overall HVAC system performance, and without the need for mobile or autonomous treatment devices, thereby providing a technically simple, safe and industrially applicable solution.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above-described embodiments are combinable.

The following dependent claims further set out particular embodiments of the disclosure.

## Claims

1. A microbiological inactivation apparatus for HVAC systems for delivering a predetermined minimum accumulated UV-C dose to circulating air, configured as a stationary module installable in-line or in bypass within an HVAC duct, the apparatus comprising:
a grille;
one or more UV-C radiation sources configured to emit ultraviolet radiation in a wavelength range effective for microbiological inactivation;
an enclosure defining an internal treatment volume for an airflow path for HVAC air;
wherein the enclosure comprises an expanding channel comprising a plurality of baffles offset from one another along the expanding direction of the channel for defining a meandering internal airflow geometry;
wherein the meandering internal airflow geometry has a cumulative internal flow length greater than the external longitudinal length of the module;
wherein the expanding channel is inclined in relation to the grille.

2. The microbiological inactivation apparatus according to the previous claim, wherein the predetermined minimum accumulated UV-C dose is at least 20 mJ/cm², calculated as a function of effective irradiance and air residence time.

3. The microbiological inactivation apparatus according to any of the previous claims, wherein the cumulative internal flow length of the airflow path is at least 1.5 times the external longitudinal length of the module.

4. The microbiological inactivation apparatus according to any of the previous claims, wherein the internal treatment volume is optically and mechanically enclosed such that UV-C irradiance measured outside the module remains below a predefined safety threshold during operation.

5. The microbiological inactivation apparatus according to any of the previous claims, wherein the apparatus is configured to operate with a pressure loss not exceeding 20% of the nominal HVAC system pressure.

6. The microbiological inactivation apparatus according to any of the previous claims, wherein at least one airflow-driving mechanism is configured to cooperate with the HVAC system to convey air through the treatment volume, preferably the airflow-driving mechanism is a blower.

7. The microbiological inactivation apparatus according to any of the previous claims, wherein the apparatus is configured to operate without physical displacement of the UV-C sources or the module during operation.

8. The microbiological inactivation apparatus according to the previous claim, wherein the meandering internal airflow geometry comprises multiple directional changes configured to increase the residence time of the air without increasing the external length of the module.

9. The microbiological inactivation apparatus according to any of the previous claims, wherein the UV-C lamps are positioned within the airflow pathway of the sinuous circuit to ensure multiple passes of the air through the UV-C radiation zone for increased disinfection efficiency.

10. The microbiological inactivation apparatus according to any of the previous claims, wherein the airflow-driving element comprises a fan or blower operable in cooperation with the HVAC system, optionally configured for bidirectional airflow depending on system requirements.

11. The microbiological inactivation apparatus according to any of the previous claims, wherein the frequency inverter is configured for control of the motor speed of the bidirectional extractor.

12. The microbiological inactivation apparatus according to any of the previous claims, wherein the effective exposure time of the air to UV-C radiation is determined exclusively by the internal geometry of the enclosure and by the volumetric airflow rate of the HVAC system, without mechanical displacement or movement of the UV-C radiation sources.

13. The microbiological inactivation apparatus according to any of the previous claims, wherein the enclosure comprises optical confinement features including overlapping panels, labyrinth seals and UV-opaque materials, such that no direct or indirect UV-C radiation is emitted outside the enclosure during operation.

14. The microbiological inactivation apparatus according to any of the previous claims, wherein the one or more UV-C radiation sources are configured to emit radiation at wavelengths from 190-250 nm, preferably the one or more UV-C radiation sources are UV-C radiation lamps.

15. The microbiological inactivation apparatus according to any of the previous claims, wherein the UV-C radiation source and motor are configured to minimize energy consumption while maintaining effective microbiological inactivation and air circulation.

16. The microbiological inactivation apparatus according to any of the previous claims, configured for continuous operation to provide ongoing microbiological disinfection of air or on-demand activation based on air quality sensors or user preferences.

17. The microbiological inactivation apparatus according to any of the previous claims, wherein the microbiological inactivation module is configured to integrate seamlessly into existing HVAC grills without requiring significant modifications to the blower or ductwork.

18. The microbiological inactivation apparatus according to any of the previous claims, wherein the sinuous circuit optimizes the use of space within the HVAC grill to accommodate high air volumes while ensuring effective pathogen inactivation.

19. The microbiological inactivation apparatus according to any of the previous claims, wherein the UV-C source inactivates airborne pathogens, allergens, and pollutants, preferably wherein the UV-C source is enclosed within a protective housing, more preferably wherein the arrangement of the UV-C source within the sinuous circuit ensures uniform and prolonged exposure of air to UV-C radiation for optimal pathogen inactivation.

20. The microbiological inactivation apparatus according to any of the previous claims, wherein the external structure of the microbiological inactivation module is compact and modular.

21. The microbiological inactivation apparatus according to any of the previous claims, wherein the bidirectional blower adapts to environmental needs by selectively operating in:
intake mode for drawing air into the HVAC system for purification; and
exhaust mode for removing contaminated air from the indoor environment.

22. The microbiological inactivation apparatus according to any of the previous claims, further comprising air quality sensors configured to monitor indoor air conditions and automatically activate the UV-C lamps and extractor based on predefined thresholds for contaminants or pathogens.
